# EUROPEAN PATENT APPLICATION

(11) **EP 1 551 069 A1**
(43) Date of publication of application: **06.07.2005**
(21) Application number: 04029359.9
(22) Date of filing: 10.12.2004
(51) Int. Cl.: H01M 4/58, H01M 4/86, H01M 4/96, C01B 31/00

(54) **Medium rate and high rate batteries**

(30) Priority: 12.12.2003 US 529317 P
(71) Applicant: Greatbatch Technologies Advanced Research Laboratories, Inc., Fremont California 94538 (US)
(72) Inventor: Ghantous, Dania, Walnut Creek California 94598 (US); Damon, Kennan O., Danville California 94526 (US); Lemkin, Jason M., Los Altos California 94024 (US)
(74) Representative: Koepe, Gerd L., Dipl.-Chem.

(57) **Abstract**

Improved submicron carbon fluoride has increased graphite content and can also have improved uniformity. The increased graphite content and/or uniformity can result in improved battery performance, for example with respect to specific capacity. Desirable battery structures provide for use with implantable medical devices. Suitable batteries can be used for high rate, medium rate, low rate or a combination of rate applications.

## Description

### CROSS REFERENCE TO RELATED APPLICATIONS

This application claims priority to U.S. Provisional Application Serial No. 60/529,317, filed on December 12, 2003.

### FIELD OF THE INVENTION

The invention relates to batteries and in particular to batteries that are suitable for medium and high rate discharge applications. The invention further relates to methods for forming medium and high rate batteries as well as corresponding electrodes.

### BACKGROUND OF THE INVENTION

Medium rate and high rate batteries can be used in various special applications in which particular performance characteristics are desired. In particular, medium rate batteries and high rate batteries can find particular usefulness in the powering of medical devices including, for example, implantable medical devices. Medium rate and high rate medical devices such as neurostimulators, pacemakers, implantable cardioverter defibrillators, and congestive heart failure devices use power sources that provide 'milliampere level electrical pulses (in the case of medium rate devices) and ampere level electrical pulses (in the case of high rate devices) to control pain, muscle movement, neurological disorders, bradycardia, tachycardia and cardiac resynchronization therapy. Existing power sources are limited in power capability therefore resulting in shorter lifetimes. Additionally, increased internal impedance at beginning and end of life results in a capacity reduction therefore shortening the rated lifetime of the device.

Lithium-based batteries have become commercially successful due to their relatively high energy density. Lithium-based batteries can be single use batteries, i.e., primary batteries, or rechargeable, i.e., secondary batteries. Suitable positive electrode materials for lithium-based batteries include materials that can intercalate lithium atoms into their lattice. The negative electrode can be lithium metal, lithium alloys or compounds that can reversibly intercalate lithium atoms into their lattice. In conventional terminology, lithium-based batteries formed from lithium metal or lithium alloy negative electrodes are referred to as lithium batteries while batteries formed with an anode (negative electrode) active material that can intercalate lithium ions are referred to as lithium ion batteries.

### SUMMARY OF THE INVENTION

In a first aspect, the invention pertains to a collection of particles comprising graphitic carbon fluoride with an average formula (CFₓ) with 1.9 ≥ x ≥ 0.6 and having an average particle diameter of no more than 1 micron. The collection of particles comprises particles having a graphitic shell with a domain thickness of at least about 3.5 nm. An electrochemical cell can be formed comprising an anode, a cathode comprising the graphitic carbon fluoride and an electrolyte activating the cathode and anode.

In another aspect, the invention pertains to a collection of particles comprising carbon fluoride with a formula of (CFₓ) with 1.9 ≥ x ≥ 0.6 and having an average particle diameter of no more than 1 micron. At least about 95 percent of the primary particles have a diameter greater than about 45 percent of the average diameter and less than about 200 percent of the average diameter.

In a further aspect, the invention pertains to a method for forming carbon fluoride, the method comprising heating carbon black particles to a temperature of at least about 2000°C and heating the particles in the presence of a fluorinating agent.

In an additional aspect, the invention pertains to a method for forming carbon fluoride particles, the method comprising reacting a flowing reactant stream comprising a carbon precursor and a fluorine precursor to form carbon fluoride particles. The reaction is driven by an electromagnetic radiation source.

Also, the invention pertains to a method for forming fluorinated carbon, the method comprising exposing carbon particles to a fluorinating agent, wherein the carbon particles were formed by laser pyrolysis.

Moreover, the invention pertains to a battery comprising a lithium based anode, a cathode comprising heat treated carbon black particles having a graphite shell having a domain thickness of at least about 3.5 nm and an electrolyte comprising lithium cations.

### BRIEF DESCRIPTION OF THE DRAWINGS

Fig. 1 is a schematic, sectional view of an embodiment of a laser pyrolysis apparatus, where the cross section is taken through the middle of the laser radiation path. The upper insert is a bottom view of the exit nozzle, and the lower insert is a top view of the injection nozzle.
Fig. 2 is a schematic, side view of a reactant delivery' apparatus for the delivery of vapor reactants to the laser pyrolysis apparatus of Fig. 1.
Fig. 3 is a perspective view of an alternative embodiment of a laser pyrolysis apparatus.
Fig. 4 is a sectional view of the inlet nozzle of the alternative laser pyrolysis apparatus of Fig. 3, the cross section being taken along the length of the nozzle through its center.
Fig. 5 is a sectional view of the inlet nozzle of the alternative laser pyrolysis apparatus of Fig. 3, the cross section being taken along the width of the nozzle through its center.
Fig. 6 is a schematic perspective view of an embodiment of an elongated reaction chamber for performing laser pyrolysis.
Fig. 7 is a perspective view of a particular embodiment of an elongated reaction chamber for performing laser pyrolysis.
Fig. 8 is a cut away, side view of the reaction chamber of Fig. 7.
Fig. 9 is a partially sectional, side view of the reaction chamber of Fig. 7, taken along line 9-9 of Fig. 7.
Fig. 10 is a fragmentary, perspective view of an embodiment of a reactant nozzle for use with the chamber of Fig. 7.
Fig. 11 is a schematic, sectional view of an apparatus for heat-treating nanoparticles, in which the section is taken through the center of the apparatus.
Fig. 12 is a schematic, perspective view of a battery of the invention.
Fig. 13 is a schematic perspective view of a multiple cathode/anode battery stacked for assembly.
Fig. 14 is a schematic sectional view of a wound battery in which the cross section is taken along a plane to expose the winds.
Fig. 15 is a schematic side view of a battery with a serpentine anode and cathode plates between the folds of the anode.
Fig. 16 is a schematic side view of a cathode with layers on opposite sides of a current collector with the layers having different electroactive materials.

### DETAILED DESCRIPTION OF THE INVENTION

The improved batteries described herein take advantage of improved rate capability and power capacities of submicron electroactive particles, and improved battery materials and compositions relate to these submicron electroactive particles. In particular, it is desirable to incorporate submicron fluorinated carbon (CFₓ) into batteries, especially for implantable medical devices, due to the ability for obtaining very high specific capacities. For the submicron carbon fluoride, another consideration is the graphitic content. Improved graphitic content is described herein for submicron carbon fluoride. In some embodiments, further advantages are gained by combining multiple cathode active compositions to take advantage of desirable characteristics of each. Improved performance characteristics can also be obtained from the incorporation of other nanoscale electroactive particles alone or in combination with additional electroactive materials within medium rate batteries or high rate batteries.

Desirable forms of submicron carbon fluoride particles can have a graphitic shell. Unfluorinated forms of carbon black with graphitic shells can also be used for electrochemical cell applications, although these materials are generally used in anodes of lithium-based batteries. The improved performance properties of the electroactive materials also provide for the use of improved processing approaches. In particular, electrode compositions can be formulated for high rate applications and combined within electrodes for medium rate applications without extensive retooling for the handling of the electrode compositions, as explained further below.

The batteries of interest generally comprise one or more anodes, one or more cathodes, and a separator between adjacent anode-cathode couples. Although solid electrolytes can be used as a separator, a liquid electrolyte is generally used for medium and high rate applications. For some of the battery chemistries described herein, a non-aqueous lithium-based electrolyte provides desired performance. While various electrode chemistries can take advantage of improved performance from nanoscale materials, lithium based batteries have the convenience of high energy density for the lithium. In some embodiments, two different cathode active materials are introduced to provide desired properties of each material. To achieve medium rate capability or high rate capability, it may be desirable to control the thickness of an electrode to influence the performance of the battery. With a selected electrode thickness, the total capacity of the resulting battery can be selected by stacking electrodes in parallel, winding the electrode, folding the electrode or the like to increase the capacity and/or to fill the battery dimensions. The discussion focuses herein on primary batteries, although some of the improved materials and processing approaches may be useful also for forming secondary, i.e., rechargeable, batteries.

While some of the electroactive materials described herein are suitable for a range of battery chemistries structures, batteries based on lithium anodes are of interest. The lithium can be metallic lithium, i.e., un-ionized Li⁰, or an alloy thereof. In other embodiments based on electrolytes with lithium ions, the anode comprises a material that can reversibly intercalate lithium ions into their lattice. The cathode is generally comprises of a particulate electroactive composition. Suitable cathode electroactive materials for lithium-based batteries include, for example, various metal chalcogenide compositions, especially metal oxides. In particular, metal vanadium oxide compositions have been identified as having high energy densities and high power densities, when used in positive electrodes for lithium-based batteries. Silver vanadium oxide has a particularly high energy density and high power densities, when used in lithium-based batteries, and has desirable properties in high rate applications. Silver vanadium oxide batteries have found particular use in the production of implantable cardiac defibrillators where the battery recharges a capacitor to deliver large pulses of energy in rapid succession. In other applications for implantable medical devices, such as medium rate applications, it is desirable to use carbon fluoride particles as the cathode active materials due to their very high energy density.

With respect to submicron particles especially for high rate applications, significantly improved performance characteristics have been observed for nanoscale silver vanadium oxide in comparison with traditional silver vanadium oxide batteries. The CFₓ submicron particles described herein can be combined with silver vanadium oxide in batteries as described further below. The synthesis of submicron metal vanadium oxide particles, in particular silver vanadium oxide particles, is described in U.S. Patent No. 6,225,007 to Horne et al., entitled "Metal Vanadium Oxide Particles" and U.S. Patent No. 6,391,494 to Reitz et al., entitled "Metal Vanadium Oxide Particles," both of which are incorporated herein by reference. Other suitable metal vanadium oxides are described in these patents including, for example, copper vanadium oxide, gold vanadium oxide and combinations thereof, such as copper silver vanadium oxide. These patents further describe the incorporation of these particles into improved batteries, especially lithium-based batteries. Improved techniques for the synthesis of metal vanadium oxides with submicron vanadium oxides as starting materials and imprbved approaches for constructing batteries from submicron metal vanadium oxide particles with the result of significantly improved high rate capacity batteries are described further in copending U.S. Application Serial No. 10/624,226, filed on July 22, 2003 to Ghantous et al., entitled "High Capacity And High Rate Batteries," incorporated herein by reference.

Other metal oxides may be useful for the formation of medical batteries, such as manganese oxides, for example, MnO₂. The formation of manganese oxide nanoparticles in various oxidation states by laser pyrolysis is described in U.S. Patent No. 6.506,493 to Kumar et al., entitled "Metal Oxide Particles," incorporated herein by reference. Vanadium oxide nanoparticles have been found to yield very high specific capacities. The formation of vanadium oxide nanoparticles, such as V₂O₅ and V₆O₁₃, is described further in U.S. Patent No. 6,106,798 to Kambe et al., entitled "Vanadium Oxide Nanoparticles, and improved battery performance with the vanadium oxide nanoparticles is described in U.S. Patent No. 6,130,007 to Bi et al., entitled "Batteries With Electroactive Nanoparticles," both of which are incorporated herein by reference.

Furthermore, carbon materials have been used as electroactive materials in lithium-based batteries. In particular, some carbon materials can effectively intercalate lithium atoms to effectuate an electrochemical reaction. In particular, graphitic carbon materials have been used in the negative electrode/anode for secondary or primary lithium ion batteries since graphitic carbon reversibly intercalates lithium. It has also been found that fluorinated graphitic carbon is a high specific capacity electroactive cathode material for lithium-based batteries. For example, U.S. Patent No. 4,681,823 to Tung et al., entitled "Lithium/Fluorinated Carbon Battery With No Voltage Delay," incorporated herein by reference, describes the formation of lithium batteries with CFₓ formed with petroleum coke. Also, the formation of lithium-based batteries with CFₓ and a solid polymer electrolyte is described in U.S. Application Pub. No. 2003/0211383 to Munshi et al., entitled "Primary Lithium Batteries," incorporated herein by reference.

Fluorinated carbon in various possible forms can be represented by (CFₓ), where x represents the degree of fluorination. Consistent with the different terminology in the art, carbon fluoride, graphite fluoride, fluorinated graphite, fluorinated carbon and comparable terms are used interchangeable without specific regard to the degree of graphite content. Generally, x falls within the range 2 > x ≥ 0. In general, improved performance can be expected for graphitic carbon, although other carbon structures may be suitable for certain applications. The use of fluorinated graphite in combination with manganese oxide is described in U.S. Patent No. 5,443,930 to Shoji et al., entitled "Nonaqueous Electrolyte Battery," incorporated herein by reference. In contrast with crystalline graphitic carbon, fluorinated fullerenes, which are not graphitic carbon since they are nonplanar, have been incorporated into the cathode of lithium-based batteries, as described in U.S. Patent Application Pub. No. 2002/0182506A to Cagle, entitled "Fullerene-Based Secondary Cell Electrodes, "incorporated herein by reference. Similarly, carbon nanotubes are not considered graphitic carbon as used herein.

While submicron carbon particles can be fluoronated following formation of the carbon particles, the incorporation of fluorine into the materials results in a significant change in the structure. Specifically, the carbon-fluorine bond results in a much larger structure relative to the corresponding pure carbon materials. Thus, fluorination of the carbon materials results in a significant expansion of the material structure. This expansion of the structure can fracture the particles and lead to irregular and un-uniform particle collections. Thus, if the fluorination can be performed during particle formation, the expansion of the particle structure can be avoided. Such a process is described further below. So fluorination of the particles during formation can result in a more desirable particle properties whether or not additional processing is performed following initial particle production.

Improved battery performance can be obtained by using submicron or nanoscale carbon fluoride (CFₓ), which may result from the increased surface area and improved lithium uptake. In addition, improved battery performance can be obtained by altering the crystal structure of the material, generally by increasing the graphitic content. Graphitic carbon has sheets of crystalline carbon stacked in layers. There are several approaches for characterizing the graphitic content. In particular, in some embodiments the submicron/nanoscale carbon is substantially graphitic, e.g., having at least 10 mole percent graphitic carbon, as determined by infrared spectroscopy. Alternatively, the submicron carbon has at least about 10 volume percent as determined by examination of an electron micrograph. In other embodiments, the submicron particles have an average graphite domain thickness of at least about 3.5 nm as determined by the examination of an electron micrograph. In contrast, carbon can be amorphous or have a diamond lattice, and carbon black particles generally have graphine layers similar to an onion, such that the material is not crystalline since there are no symmetry axes, although carbon black particles can have small graphitic domains.

The submicron elemental carbon particles and carbon fluoride particles can be formed, for example, by laser pyrolysis, either directly or with additional processing. Laser pyrolysis is generally a misnomer since intense non-laser light sources can serve as the energy source and since the process is not a pyrolysis in the sense of a purely thermal pyrolysis process.

A basic feature of successful application of laser pyrolysis for the production of carbon particles, carbon fluoride particles or the like is production of a reactant stream containing an appropriate precursor and a radiation absorber. Generally, some iron is introduced to serve as a catalyst. An intense light beam, such as a laser beam, pyrolyzes the reactant stream. While a laser beam is a convenient energy source, other intense light sources can be used in laser pyrolysis. Laser pyrolysis provides for formation of phases of materials that are difficult to form under thermodynamic equilibrium conditions. As the reactant stream leaves the light beam, the particles are rapidly quenched. The production of submicron carbon particles by laser pyrolysis is described further in Bi et al., "Nanoscale Carbon Blacks Produced By CO₂ Laser Pyrolysis," Material Research Society, Symposium Proceedings, Vol. 286, pp 161-167 (1993), incorporated herein by reference. It was further found that heat treatment at temperatures of 2,000°C or 2,850°C in an inert gas environment increased the graphitic content of the particles without resulting in any significant sintering of the particles. Laser pyrolysis is generally a very desirable approach to particle formation since the primary particles generally have little if any hard fusing such that the primary particles are highly dispersible and such that the nanoscale properties can be fully used to an advantage.

The introduction of fluorine generally into product particles formed by laser pyrolysis is described in U.S. Patent Application Pub. No. 2003/0118841 to Horne et al., entitled "Optical Materials And Optical Devices," incorporated herein by reference. In general, a fluorine source can be introduced that is compatible with a carbon source. For example, gaseous carbon tetrafluoride can be introduced into the reactant stream of the laser pyrolysis apparatus. Selecting the appropriate reaction conditions within the laser pyrolysis reactor, such as by altering the reactant flow rate can vary the graphitic composition, the laser intensity, and the relative concentrations of the reactants. This includes, for example, varying the inert diluent gases within the reactant flow. Additional processing after production of the particles can be used to increase the desirable properties of the particles, such as the graphite content of carbon particles, the crystallinity and purity of metal oxide particles and the like. Heat treatments to improve the characteristics of particles is described further below.

In alternative embodiments, the fluorination of the submicron, graphitic carbon particles are performed after the formation of the particles. In particular, fluorine gas (F₂) and/or HF can be used to fluorinate the submicron carbon particles. Appropriate heating, generally in the range from about 150°C to about 400°C drives the fluorination reaction. The degree of fluorination can be selected through the control of the fluorination conditions. For example, U.S. Patent No. 4,423,261 to Watanabe et al., entitled "Process For Producing A Graphite Fluoride Comprising Mainly Polydicarbon Monofluoride Represented By The Formula (C₂F)ₙ," incorporated herein by reference, described fluorination of graphitic material using F₂ in the presence of a metal fluoride with an alkali metal, an alkali earth metal or a first row transition metal. Another processing approach for forming fluorinated graphite is described in U.S. Patent No. 4,795,624 to Nalewajek, entitled "Low Temperature Synthesis Of Graphite Based Carbon Fluoride And Carbon Fluoride Chloride," incorporated herein by reference. The formation of fluorinated graphite using a combination of IF₅, F₂ and HF is described in U.S. Patent No. 6,358,649 to Yazami et al., entitled "Carbons Containing Fluorine, Method Of Preparation Thereof And Use As Electrode Material," incorporated herein by reference. The fluorination of carbon blacks is described in U.S. Patent No. 4,855,121 to Metzger, entitled "Continuous Process For The Preparation of Carbon Polymonofluoride And Apparatus Therefor [sic]," and U.S. Patent No. 4,859,444 to Kita et al., entitled "Method of Producing Ultrafine Particles of Graphite Fluoride," both of which are incorporated herein by reference. These approaches for the fluorination of carbon particles can be adapted for the fluorination of carbon particles formed by laser pyrolysis.

In general, desired CFₓ submicron particles can have an average diameter for the particles of less than about 1 micron. Furthermore, the particles generally have very high size uniformity, which can result in improved performance for many applications. Furthermore, it can be advantageous to provide a combination of cathode active materials within a battery. Thus, advantages of different cathode active materials can be combined for more versatile application of a battery. For example, it may be desirable to have a battery that can provide high rate pulsed discharge along with very high capacity for low current constant drain.

Thus, it may be desirable to have a high rate cathode material such as silver vanadium oxide combined with a high capacity material, such as CFₓ. One or more of these materials may be in the form of submicron particles. In general, a cathode for a medical battery can comprise CFₓ with another electroactive material, for example, silver vanadium oxide, copper vanadium oxide, vanadium oxide, silver oxide, manganese oxide and a combination thereof, such as copper silver vanadium oxide. The two or more different cathode active compositions can be placed within a single electrode structure as a blend of electroactive materials, within different layers of an electrode structure, in different electrode structures coupled to the same anode system, and/or within different electrode structures coupled to two different anode systems within a single case, although a sandwich structure with a current collector separating two different cathode active materials is of particular interest as described further below.

The use of the improved nanoparticle electroactive materials described herein provides for the formation of the electrode compositions with improved properties. In particular, the powders of the nanoparticles can be combined with slightly larger amounts of binder due to the improved capacity. With appropriate blending, the resulting electrode compositions can be more uniform while having better cohesion and adhesion. As a result of the improved properties, the electrode compositions can have significantly improved handling properties.

By way of example, submicron carbon fluoride prepared by laser pyrolysis with or without additional processing results in small particle sizes with a narrow particle size distribution. The small particle size and narrow distribution allows for higher pressed densities, which control the shape of the discharge profile and enhance the cell's delivered capacity. Additionally, the homogeneous submicron carbon fluoride-based cathodes deliver lower internal impedance at beginning of life therefore maintaining rated capacities and enhancing longevity. Small and homogeneous particle size combined with low internal impedance increase the current capability of the submicron carbon fluoride-based cathodes providing higher current capability while maintaining delivered capacity. These performance benefits allow the use of more sophisticated functionality in high rate and medium rate devices.

As noted above, a plurality of electroactive materials can be combined for positive electrodes in a single battery or device. For example, metal vanadium oxide can be combined with carbon fluoride. The metal vanadium oxide, such as silver vanadium oxide, is particularly desirable for high rate applications while the carbon fluoride are particle desirable for medium rate or low rate applications that can benefit from the high capacity properties of these materials. When used in combination, the carbon fluoride can be used to regenerate the silver vanadium oxide between pulses in pulse operation to take advantage of the high capacity of the carbon fluoride and the high rate capability of the silver vanadium oxide.

Various layered structures can be effectively used to get the advantages from a plurality of cathode active materials. For example, cathode layers formed from two different electroactive materials can be placed on opposite sides of a current collector. Thus, the high rate discharge material can be placed closer to the corresponding anode to further reduce the diffusion times of ions from the electrolyte. Other structures can also be effective for specific applications. As another example, a sandwich structure has two different cathode layers that are sandwiched between two current collectors, as described further in U.S. Patent No. 6,551,747 to Gan, entitled "Sandwich Cathode Design For Alkali Metal Electrochemical Cell With High Discharge Rate Capability," incorporated herein by reference.

The improved materials described herein can effective used to form a range of battery structures. A range of structures can take advantage of the improved properties of the carbon fluoride particles described herein. The improved carbon fluoride should provide improved discharge capacities that are particularly desirable for applications such as for implantable medical devices since a longer life can decrease reoperation rates or provide for smaller devices that are easier to implant.

### A. Particle Production Using Laser Pyrolysis

As described above, laser pyrolysis is a valuable tool for the production of submicron and nanoscale particles of interest. The particles can be directly'formed as materials of interest, and/or they can be subjected to additional processing, such as a heat treatment, to form desired materials. With respect to some medical battery applications, carbon particles and fluorinated. carbon particles can be of interest.

The reaction conditions determine the qualities of the particles produced by laser pyrolysis. The reaction conditions for laser pyrolysis can be controlled relatively precisely in order to produce particles with desired properties. The appropriate reaction conditions to produce a certain type of particles generally depend on the design of the particular apparatus. Some general observations on the relationship between reaction conditions and the resulting particles can be made for particle production using laser pyrolysis.

Increasing the light power results in increased reaction temperatures in the reaction region as well as a faster quenching rate. A rapid quenching rate tends to favor production of high-energy phases, which may not be obtained with processes near thermal equilibrium. Similarly, increasing the chamber pressure also tends to favor the production of higher energy structures. Also, increasing the concentration of the reactant serving as the oxygen source in the reactant stream favors the production of particles with increased amounts of oxygen.

Reactant flow rate and velocity of the reactant gas stream are inversely related to particle size so that increasing the reactant gas flow rate or velocity tends to result in smaller particle sizes. Light power also influences particle size with increased light power favoring larger particle formation for lower melting materials and smaller particle formation for higher melting materials. Also, the growth dynamics of the particles have a significant influence on the size of the resulting particles. In other words, different forms of a product compound have a tendency to form different size particles from other phases under relatively similar conditions. Similarly, in multiphase regions at which populations of particles with different compositions are formed, each population of particles generally has its own characteristic narrow distribution of particle sizes.

Laser pyrolysis has become the standard terminology of reactions driven by a intense light radiation with rapid quenching of product after leaving a narrow reaction region defined by the light. The reaction is not a pyrolysis in the sense of a thermal pyrolysis. The laser pyrolysis reaction is not thermally driven by the exothermic combustion of the reactants. In fact, the "laser pyrolysis" reaction can be conducted under conditions where no visible flame is observed from the reaction.

Laser pyrolysis has been performed generally with gas/vapor phase reactants. Many precursor compounds can be delivered into the reaction chamber as a gas. Appropriate precursor compounds for gaseous delivery generally include compounds with reasonable vapor pressures, i.e., vapor pressures sufficient to get desired amounts of precursor gas/vapor into the reactant stream. The vessel holding liquid or solid precursor compounds can be heated to increase the vapor pressure of the precursor, if desired. Solid precursors generally are heated to produce a sufficient vapor pressure. The reactant stream can comprise gaseous reactants, which can be in addition to vapor reactants delivered form a liquid or solid precursor reservoir.

A carrier gas can be bubbled through a liquid precursor to facilitate delivery of a desired amount of precursor vapor. Similarly, a carrier gas can be passed over the solid precursor to facilitate delivery of the precursor vapor. Suitable iron precursors for catalyst delivery for carbon production include, for example, iron pentacarbonyl, Fe(CO)₅.

The use of exclusively gas phase reactants is somewhat limiting with respect to the types of precursor compounds that can be used conveniently. Thus, techniques have been developed to introduce aerosols containing reactant precursors into laser pyrolysis chambers. Improved aerosol delivery apparatuses for reaction systems are described further in U.S. Patent No. 6,193,936 to Gardner et al., entitled "Reactant Delivery Apparatuses," incorporated herein by reference. One or more aerosol reactants can be combined with one or more vapor reactants, as appropriate. For convenience, vapor reactants refer to compositions delivered as a vapor into the reaction chamber regardless of whether or not the compositions are gases, liquids or solids at room temperature and pressure.

A variety of carbon precursors are suitable including, for example, gaseous, liquid and solid compositions. Suitable precursors include, for example, ethylene and benzene, which can be delivered in vapor form into the reaction chamber. Ethylene can be desirable due to its strong absorption of infrared light from a CO₂ laser.

For relevant embodiments, suitable reactants serving as an oxygen source include, for example, O₂, CO, H₂O, CO₂, O₃ and mixtures thereof. Molecular oxygen can be supplied as air. The secondary reactant compound should not react significantly with the other precursors prior to entering the reaction zone since this generally would result in the formation of large particles. If the reactants are spontaneously reactive, the different precursors can be delivered in separate nozzles into the reaction chamber such that they are combined just prior to reaching the light beam. If the other precursor includes oxygen, a separate reactant may not be needed to supply oxygen.

Laser pyrolysis can be performed with a variety of optical frequencies, using either a laser or other strong light source. Suitable light sources operate in the infrared portion of the electromagnetic spectrum. CO₂ lasers are particularly convenient sources of light. Infrared absorbers for inclusion in the reactant stream include, for example, C₂H₄, isopropyl alcohol, NH₃, SF₆, SiH₄ and O₃. O₃ can act as both an infrared absorber and as an oxygen source. For embodiments based on the formation of carbon particles, C₂H₄ can serve as a carbon source as well as an infrared absorber. The radiation absorber, such as the infrared absorber, absorbs energy from the radiation beam and distributes the energy to the other reactants to drive the pyrolysis.

Preferably, the energy absorbed from the light beam increases the temperature at a tremendous rate, many times the rate that heat generally would be produced by exothermic reactions under controlled condition. While the process generally involves nonequilibrium conditions, the temperature can be described approximately based on the energy in the absorbing region. The laser pyrolysis process is qualitatively different from the process in a combustion reactor where an energy source initiates a reaction, but the reaction is driven by energy given off by an exothermic reaction.

An inert shielding gas can be used to reduce the amount of reactant and product molecules contacting the reactant chamber components. Inert gases can also be introduced into the reactant stream as a carrier gas and/or as a reaction moderator. Appropriate inert shielding gases include, for example, Ar, He and N₂.

An appropriate laser pyrolysis apparatus generally includes a reaction chamber isolated from the ambient environment. A reactant inlet connected to a reactant delivery apparatus produces a reactant stream through the reaction chamber. A light beam path intersects the reactant stream at a reaction zone. The reactant/product stream continues after the reaction zone to an outlet, where the reactant/product stream exits the reaction chamber and passes into a collection apparatus. Generally, the light source, such as a laser, is located external to the reaction chamber, and the light beam enters the reaction chamber through an appropriate window.

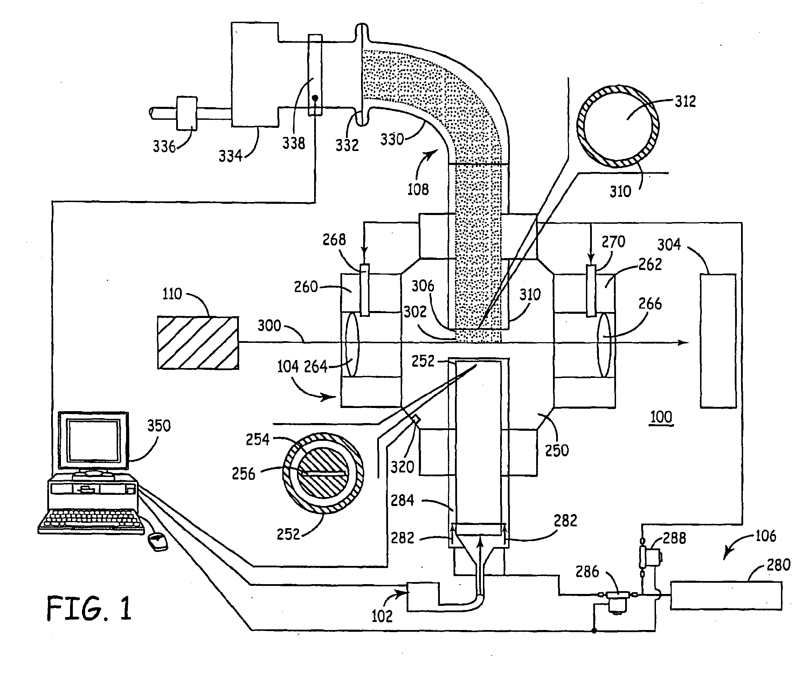

Referring to Fig. 1, a particular embodiment 100 of a laser pyrolysis system involves a reactant delivery apparatus 102, reaction chamber 104, shielding gas delivery apparatus 106, collection apparatus 108 and light source 110. A first reaction delivery apparatus described below can be used to deliver exclusively gaseous reactants.

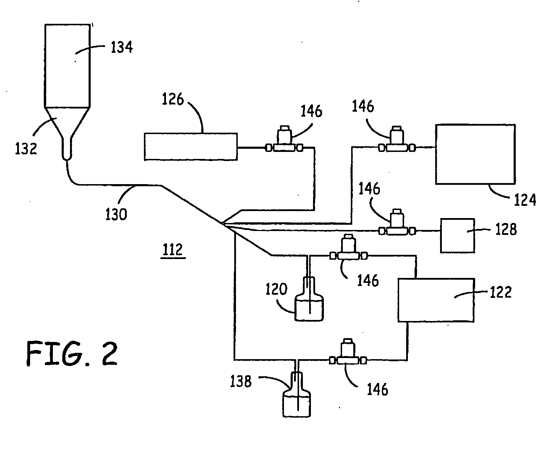

Referring to Fig. 2, a first embodiment 112 of reactant delivery apparatus 102 comprises a source 120 of a precursor compound. For liquid or solid reactants, a carrier gas from one or more carrier gas sources 122 can be introduced into precursor source 120 to facilitate delivery of the reactant. Precursor source 120 can be a liquid holding container, a solid precursor delivery apparatus or other suitable container. The carrier gas from carrier gas source 122 can be either an infrared absorber and/or an inert gas.

The gases from precursor source 120 are mixed with gases from infrared absorber source 124, inert gas source 126 and/or secondary reactant source 128 by combining the gases in a single portion of tubing 130. The gases are combined a sufficient distance from reaction chamber 104 such that the gases become well mixed prior to their entrance into reaction chamber 104. The combined gas in tube 130 passes through a duct 132 into channel 134, which is in fluid communication with reactant inlet 206.

A second reactant can be supplied from second reactant source 138, which can be a liquid reactant delivery apparatus, a solid reactant delivery apparatus, a gas cylinder or other suitable container or containers. As shown in Fig. 2, second reactant source 138 delivers a second reactant to duct 132 by way of tube 130. Alternatively, mass flow controllers 146 can be used to regulate the flow of gases within the reactant delivery system of Fig. 2. The second reactant can be delivered through a second duct for delivery into the reactant chamber through a second channel such that the reactants do not mix until they are in the reaction chamber.

As noted above, the reactant stream can comprise one or more aerosols. The aerosols can be formed within reaction chamber 104 or outside of the reaction chamber prior to injection into reaction chamber 104. If the aerosols are produced prior to injection into reaction chamber 104, the aerosols can be introduced through reactant inlets comparable to those used for gaseous reactants, such as reactant inlet 134 in Fig. 2.

Referring to Fig. 1, the reaction chamber 104 includes a main chamber 250. Reactant supply system 102 connects to the main chamber 250 at injection nozzle 252. Reaction chamber 104 can be heated to a surface temperature above the dew point of the mixture of reactants and inert components at the pressure in the apparatus.

The end of injection nozzle 252 has an annular opening 254 for the passage of inert shielding gas, and a reactant inlet 256 (left lower insert) for the passage of reactants to form a reactant stream in the reaction chamber. Reactant inlet 256 preferably is a slit, as shown in the lower inserts of Fig. 1. Annular opening 254 can have, for example, a diameter of about 1.5 inches and a width along the radial direction from about 1/8 in to about 1/16 in. The flow of shielding gas through annular opening 254 helps to prevent the spread of the reactant gases and product particles throughout reaction chamber 104.

Tubular sections 260, 262 are located on either side of injection nozzle 252. Tubular sections 260, 262 include ZnSe windows 264, 266, respectively. Windows 264, 266 can be about 1 inch in diameter. Windows 264, 266 can be cylindrical lenses, for example, with a focal length equal to the distance between the center of the chamber to the surface of the lens to focus the light beam to a point just below the center of the nozzle opening. Windows 264, 266 can have an antireflective coating. Appropriate ZnSe lenses are available from Laser Power Optics, San Diego, California. Tubular sections 260, 262 provide for the displacement of windows 264, 266 away from main chamber 250 such that windows,264, 266 are less likely to be contaminated by reactants and/or products. Window 264, 266 are displaced, for example, about 3 cm from the edge of the main chamber 250.

Windows 264, 266 are sealed with a rubber o-ring to tubular sections 260, 262 to prevent the flow of ambient air into reaction chamber 104. Tubular inlets 268, 270 provide for the flow of shielding gas into tubular sections 260, 262 to reduce the contamination of windows 264, 266. Tubular inlets 268, 270 are connected to shielding gas delivery apparatus 106.

Referring to Fig. 1, shielding gas delivery system 106 includes inert gas source 280 connected to an inert gas duct 282. Inert gas duct 282 flows into annular channel 284 leading to annular opening 254. A mass flow controller 286 regulates the flow of inert gas into inert gas duct 282. If reactant delivery system 112 of Fig. 2 is used, inert gas source 126 can also function as the inert gas source for duct 282, if desired. Referring to Fig. 1, inert gas source 280 or a separate inert gas source can be used to supply inert gas to tubes 268, 270. A mass flow controller 288 preferably controls flow to tubes 268, 270.

Light source 110 is aligned to generate a light beam 300 that enters window 264 and exits window 266. Windows 264, 266 define a light path through main chamber 250 intersecting the flow of reactants at reaction zone 302. After exiting window 266, light beam 300 strikes power meter 304, which also acts as a beam dump. An appropriate power meter is available from Coherent Inc., Santa Clara, CA. Light source 110 can be a laser or an intense conventional light source such as an arc lamp. For example, light source 110 can be an infrared laser, especially a CW CO₂ laser such as an 1800-watt maximum power output laser available from PRC Corp., Landing, NJ.

Reactants passing through reactant inlet 256 in injection nozzle 252 initiate a reactant stream. The reactant stream passes through reaction zone 302, where reaction involving the metal precursor compounds takes place. Heating of the gases in reaction zone 302 is extremely rapid, roughly on the order of 10⁵ degree C/sec depending on the specific conditions. The reaction is rapidly quenched upon leaving reaction zone 302, and particles 306 are formed in the reactant/ product stream. The nonequilibrium nature of the process allows for the production of nanoparticles with a highly uniform size distribution and structural homogeneity.

The path of the reactant stream continues to collection nozzle 310. Collection nozzle 310 has a circular opening 312, as shown in the upper insert of Fig. 1. Circular opening 312 feeds into collection system 108.

The chamber pressure is monitored with a pressure gauge 320 attached to the main chamber. The preferred chamber pressure for the production of the desired oxides generally ranges from about 80 Torr to about 650 Torr.

Collection system 108 can comprise a curved channel 330 leading from collection nozzle 310. Because of the small size of the particles, the product particles follow the flow of the gas around curves. Collection system 108 includes a filter 332 within the gas flow to collect the product particles. Due to curved section 330, the filter is not supported directly above the chamber. A variety of materials such as Teflon® (polytetrafluoroethylene), glass fibers and the like can be used for the filter as long as the material is inert and has a fine enough mesh to trap the particles. Suitable materials for the filter include, for example, a glass fiber filter from ACE Glass Inc., Vineland, NJ and cylindrical Nomex® filters from AF Equipment Co., Sunnyvale, CA.

Pump 334 is used to maintain collection system 108 at a selected pressure. It may be desirable to flow the exhaust of the pump through a scrubber 336 to remove any remaining reactive chemicals before venting into the atmosphere.

The pumping rate is controlled by either a manual needle valve or an automatic throttle valve 338 inserted between pump 334 and filter 332. As the chamber pressure increases due to the accumulation of particles on filter 332, the manual valve or the throttle valve can be adjusted to maintain the pumping rate and the corresponding chamber pressure.

A computer 350 controls the apparatus. Generally, the computer controls the light source and monitors the pressure in the reaction chamber. The computer can be used to control the flow of reactants and/or the shielding gas.

The reaction can be continued until sufficient particles are collected on filter 332 such that pump 334 can no longer maintain the desired pressure in the reaction chamber 104 against the resistance through filter 332. When the pressure in reaction chamber 104 can no longer be maintained at the desired value, the reaction is stopped, and filter 332 is removed. With this embodiment, about 1-300 grams of particles can be collected in a single run before the chamber pressure can no longer be maintained. A single run generally can last up to about 10 hours depending on the reactant delivery system, the type of particle being produced and the type of filter being used.

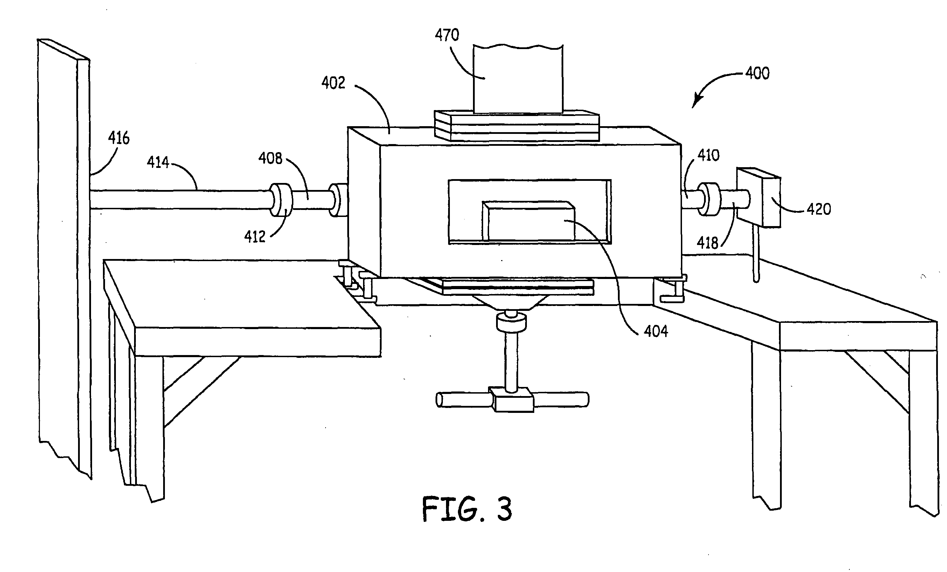

An alternative embodiment of a laser pyrolysis apparatus is shown in Fig. 3. Laser pyrolysis apparatus 400 includes a reaction chamber 402. The reaction chamber 402 has a shape of a rectangular parallelepiped. Reaction chamber 402 extends with its longest dimension along the laser beam. Reaction chamber 402 has a viewing window 404 at its side, such that the reaction zone can be observed during operation.

Reaction chamber 402 has tubular extensions 408, 410 that define an optical path through the reaction chamber. Tubular extension 408 is connected with a seal to a cylindrical lens 412. Tube 414 connects laser 416 or other optical source with lens 412. Similarly, tubular extension 410 can be connected with a seal to tube 418, which further leads to beam dump/light meter 420. The entire light path from laser 416 to beam dump 420 can be enclosed.

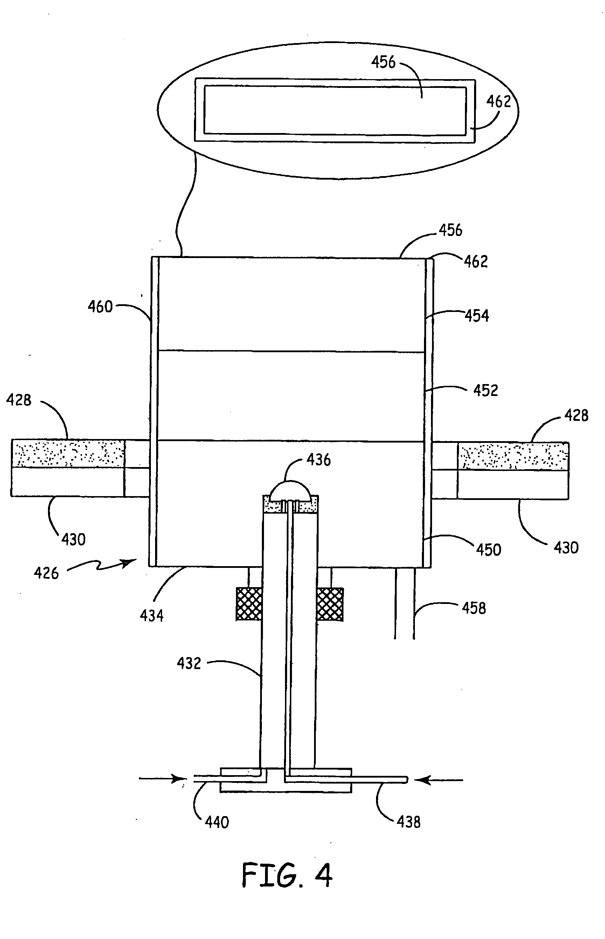

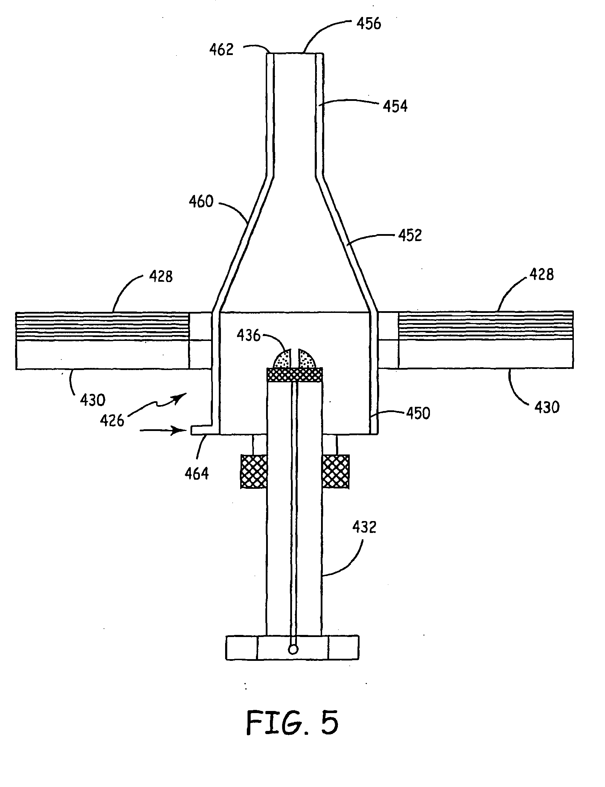

Inlet nozzle 426 connects with reaction chamber 402 at its lower surface 428. Inlet nozzle 426 comprises a plate 430 that bolts into lower surface 428 to secure inlet nozzle 426. Referring to sectional views in Figs. 4 and 5, inlet nozzle 426 includes an inner nozzle 432 and an outer nozzle 434. Inner nozzle 432 can have a twin orifice internal mix atomizer 436 at the top of the nozzle. Suitable gas atomizers are available from Spraying Systems, Wheaton, IL. The twin orifice internal mix atomizer 436 has a fan shape to produce a thin sheet of aerosol and gaseous precursors. Liquid is fed to the atomizer through tube 438, and gases for introduction into the reaction chamber are fed to the atomizer through tube 440. Droplet formation is assisted by interaction with the gas.

Outer nozzle 434 includes a chamber section 450, a funnel section 452 and a delivery section 454. Chamber section 450 holds the atomizer of inner nozzle 432. Funnel section 452 directs the aerosol and gaseous precursors into delivery section 454. Delivery section 450 leads to an about 3 inch by 0.5 inch rectangular outlet 456, shown in the insert of Fig. 4. Outer nozzle 434 includes a drain 458 to remove any liquid that collects in the outer nozzle. Outer nozzle 434 is covered by an outer wall 460 that forms a shielding gas opening 462 surrounding outlet 456. Inert gas is introduced through inlet 464.

Referring to Fig. 3, exit nozzle 470 connects to apparatus 400 at the top surface of reaction chamber 402. Exit nozzle 470 leads to filter chamber 472. Filter chamber 472 connects with pipe 474, which leads to a pump. A cylindrical filter is mounted at the opening to pipe 474. Suitable cylindrical filters are described above.

Another alternative design of a laser pyrolysis apparatus has been described in U.S. Patent 5,958,348 to Bi et al., entitled "Efficient Production of Particles by Chemical Reaction," incorporated herein by reference. This alternative design is intended to facilitate production of larger scale quantities of particles by laser pyrolysis. Additional embodiments and other appropriate features for commercial capacity laser pyrolysis apparatuses are described in copending and commonly assigned U.S. Application Serial No. 09/362,631 to Mosso et al., entitled "Particle Production Apparatus," incorporated herein by reference.

In one particular embodiment of a commercial capacity laser pyrolysis apparatus, the reaction chamber and reactant inlet are elongated significantly along the light beam to provide for an increase in the throughput of reactants and products. The original design of the apparatus was based in particular on the introduction of gaseous reactants. The embodiments described above for the delivery of aerosol reactants can be adapted for the elongated reaction chamber design. Additional embodiments for the introduction of an aerosol with one or more aerosol generators into an elongated reaction chamber is described in U.S. Patent No. 6,193,936 to Gardner et al., entitled "Reactant Delivery Apparatuses," incorporated herein by reference.

In general, the laser pyrolysis apparatus with the elongated reaction chamber and reactant inlet is designed to reduce contamination of the chamber walls, to increase the production capacity and to make efficient use of resources. To accomplish these objectives, the elongated reaction chamber provides for an increased throughput of reactants and products without a corresponding increase in the dead volume of the chamber. The dead volume of the chamber can become contaminated with unreacted compounds and/or reaction products. Furthermore, an appropriate flow of shielding gas confines the reactants and products within a flow stream through the reaction chamber. The high throughput of reactants makes efficient use of the light energy.

The improved reaction systems comprise a collection apparatus to remove the nanoparticles from the reactant stream. The collection system can be designed to collect particles in a batch mode with the collection of a large quantity of particles prior to terminating production. A filter or the like can be used to collect the particles in batch mode. One embodiment suitable for batch collection is described further below. Alternatively, the collection system can be designed to run in a continuous production mode by switching between different particle collectors within the collection apparatus or by providing for removal of particles without exposing the collection system to the ambient atmosphere. A particular embodiment of a collection apparatus for continuous particle production is described in U.S. Patent No. 6,270,732 to Gardner et al., entitled "Particle Collection Apparatus And Associated Methods," incorporated herein by reference. This collection apparatus can similarly be used with other particle production apparatuses, such as the laser pyrolysis apparatuses described above with respect to Figs. 1 and 3.

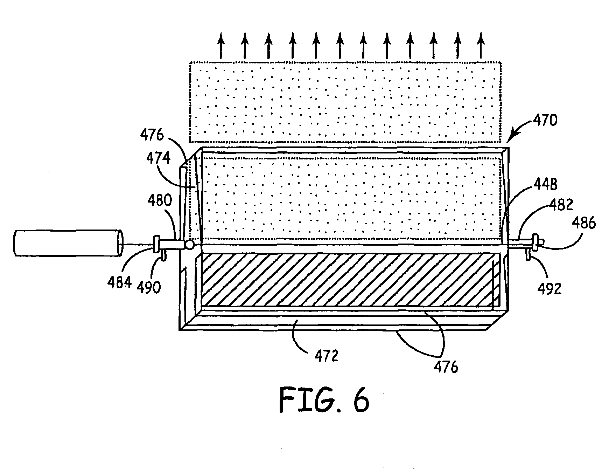

The design of a high throughput reaction chamber 470 is shown schematically in Fig. 6. A reactant inlet 472 leads to main chamber 474. Reactant inlet 472 conforms generally to the shape of main chamber 474. Main chamber 474 includes an outlet 476 along the reactant/product stream for removal of particulate products, any unreacted gases and inert gases. Shielding gas inlets 478 are located on both sides of reactant inlet 472. Shielding gas inlets are used to form a blanket of inert gases on the sides of the reactant stream to inhibit contact between the chamber walls and the reactants or products. The dimensions of elongated reaction chamber 474 and reactant inlet 472 can be designed for high efficiency particle production. Reasonable dimensions for reactant inlet 472 for the production of ceramic nanoparticles, when used with a 1800 watt CO₂ laser, are from about 5 mm to about 1 meter.

Tubular sections 480, 482 extend from the main chamber 474. Tubular sections 480, 482 hold windows 484, 486 to define a light beam path 488 through the reaction chamber 470. Tubular sections 480, 482 can include inert gas inlets 490, 492 for the introduction of inert gas into tubular sections 480, 482.

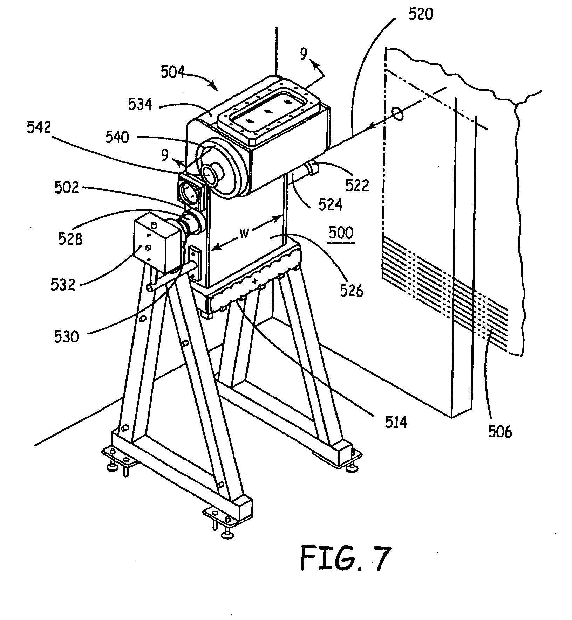

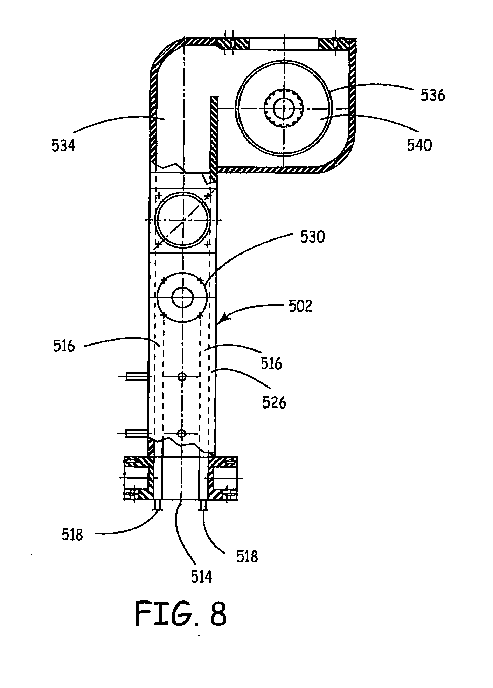

Referring to Figs. 7 to 9, a specific embodiment of a laser pyrolysis reaction system 500 with aerosol reactant delivery comprises reaction chamber 502, a particle collection system 504, laser 506 and a reactant delivery system 508. Reaction chamber 502 comprises reactant inlet 514 at the bottom of reaction chamber 502 where reactant delivery system 508 connects with reaction chamber 502. In this embodiment, the reactants are delivered from the bottom of the reaction chamber while the products are collected from the top of the reaction chamber. The configuration can be reversed with the reactants supplied from the top and product collected from the bottom.

Shielding gas conduits 516 are located on the front and back of reactant inlet 514. Inert gas is delivered to shielding gas conduits 516 through ports 518. The shielding gas conduits direct shielding gas along the walls of reaction chamber 502 to inhibit association of reactant gases or products with the walls.

Reaction chamber 502 is elongated along one dimension. denoted in Fig. 7 by "w". A laser beam path 520 enters the reaction chamber through a window 522 displaced along a tube 524 from the main chamber 526 and traverses the elongated direction of reaction chamber 502. The laser beam passes through tube 528 and exits window 530. In one embodiment, tubes 524 and 528 displace windows 522 and 530 about 11 inches from the main chamber. The laser beam terminates at beam dump 532. In operation, the laser beam intersects a reactant stream generated through reactant inlet 514.

The top of main chamber 526 opens into particle collection system 504. Particle collection system 504 comprises outlet duct 534 connected to the top of main chamber 526 to receive the flow from main chamber 526. Outlet duct 534 carries the product particles out of the plane of the reactant stream to a cylindrical filter 536. Filter 536 has a cap 538 on one end. The other end of filter 536 is fastened to disc 540. Vent 542 is secured to the center of disc 540 to provide access to the center of filter 536. Vent 542 is attached by way of ducts to a pump. Thus, product particles are trapped on filter 536 by the flow from the reaction chamber 502 to the pump. Suitable pumps were described above. Suitable pumps include, for example, an air cleaner filter for a Saab 9000 automobile (Purilator part A44-67), which is wax impregnated paper with Plasticol or polyurethane end caps.

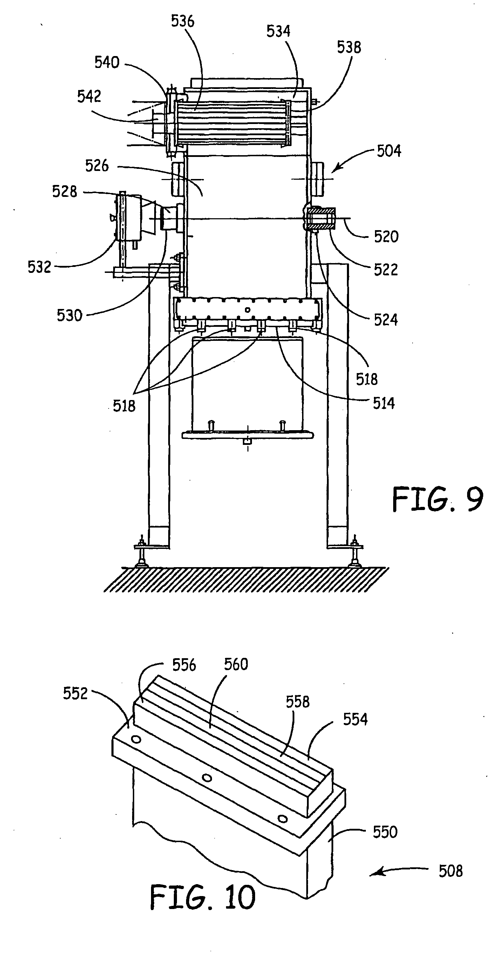

In one embodiment, reactant delivery system 508 comprises a reactant nozzle 550, as shown in Fig. 10. Reactant nozzle 550 can comprise an attachment plate 552. Reactant nozzle 550 attaches at reactant inlet 514 with attachment plate 552 bolting to the bottom of main chamber 526. In one embodiment, nozzle 550 has four channels that terminate at four slits 554, 556, 558, 560. Slits 558 and 560 can be used for the delivery of vanadium precursors and other desired components of the reactant stream. Slits 554, 556 can be used for the delivery of inert shielding gas. If a secondary reactant is spontaneously reactive with the vanadium precursor, it can be delivered also through slits 554, 556. One apparatus used for the production of vanadium oxide particles had dimensions for slits 554, 556, 558, 560 of 3 inches by 0.04 inches.

### B. Heat Processing

Significant properties of submicron and nanoscale carbon and/or carbon fluoride particles can be modified through heat processing. Suitable starting materials for the heat treatment include, for example, particles produced by laser pyrolysis. In addition, particles used as starting material for a heat treatment process can have been subjected to one or more prior heating steps under different conditions. For the heat processing of particles formed by laser pyrolysis, the additional heat processing can improve the crystallinity, remove contaminants, change the crystal structure, and/or alter the stoichiometry, for example, by fluorinating the particles.

The starting materials generally can be particles of any size and shape, although submicron and nanoscale particles are starting materials of particular interest. The nanoscale particles have an average diameter of less than about 1000 nm and in some embodiments from about 2 nm to about 500 nm, and in further embodiments from about 5 nm to about 150 nm. A person or ordinary skill in the art will recognize that additional ranges within the explicit ranges of average particle size are contemplated and are within the present disclosure. Suitable nanoscale starting materials can be produced by laser pyrolysis. Further properties of particles formed by laser pyrolysis are described below.

The particles can be heated in an oven or the like to provide generally uniform heating. The processing conditions generally are mild, such that significant amounts of particle sintering do not occur. Thus, the temperature of heating can be low relative to the melting point of the starting material and the product material.

The atmosphere over the particles can be static, or gases can be flowed through the system. The atmosphere for the heating process can be an oxidizing atmosphere, a reducing atmosphere or an inert atmosphere. In particular, for conversion of amorphous particles to crystalline particles or from one crystalline structure to a different crystalline structure of essentially the same stoichiometry, the atmosphere generally can be inert.

Appropriate oxidizing gases include, for example, O₂, O₃, CO, CO₂, and combinations thereof. The O₂ can be supplied as air. Reducing gases include, for example, H₂. Oxidizing gases or reducing gases optionally can be mixed with inert gases such as Ar, He and N₂. When inert gas is mixed with the oxidizing/reducing gas, the gas mixture can comprise from about 1 percent oxidizing/reducing gas to about 99 percent oxidizing/reducing gas, and in some embodiments from about 5 percent oxidizing/reducing gas to about 99 percent oxidizing/reducing gas. Alternatively, essentially pure oxidizing gas, pure reducing gas or pure inert gas can be used, as desired. Care must be taken with respect to the prevention of explosions when using highly concentrated reducing gases.

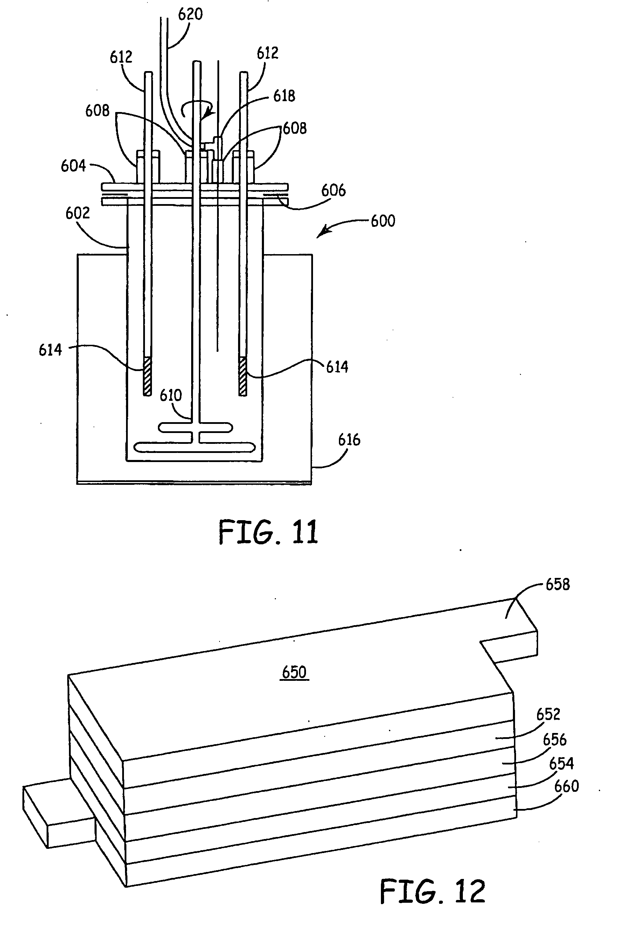

A variety of ovens or the like can be used to perform the heating. An example of an apparatus 600 to perform this processing is displayed in Fig. 11. Apparatus 600 comprises a jar 602, which can be made from glass or other inert material, into which the particles are placed. Suitable glass reactor jars are available from Ace Glass (Vineland, NJ). For higher temperatures alloy jars can be used to replace the glass jars. The top of glass jar 602 is sealed to a glass cap 604, with Teflon® gasket 606 between jar 602 and cap 604. Cap 604 can be held in place with one or more clamps. Cap 604 includes a plurality of ports 608, each with a Teflon® bushing. A multiblade stainless steel stirrer 610 can be inserted through a central port 608 in cap 604. Stirrer 610 is connected to a suitable motor.

One or more tubes 612 are inserted through ports 608 for the delivery of gases into jar 602. Tubes 612 can be made from stainless steel or other inert material. Diffusers 614 can be placed at the tips of tubes 612 to disburse the gas within jar 602. A heater/furnace 616 generally can be placed around jar 602. Suitable resistance heaters are available from Glas-col (Terre Haute, IN). One port can include, for example, a T-connection 618. The temperature within jar 602 can be measured with a thermocouple 618 inserted through T-connection 618. T-connection 618 can be further connected to a vent 620. Vent 620 provides for the venting of gas circulated through jar 602. Vent 620 can be vented to a fume hood or alternative ventilation equipment.

In some embodiments, desired gases are flowed through jar 602. Tubes 612 generally are connected to an oxidizing gas source, reducing gas source and/or an inert gas source. Oxidizing gas, reducing gas, inert gas or a combination thereof to produce the desired atmosphere is placed within jar 602 from the appropriate gas source(s). Various flow rates can be used. The flow rate can be, for example, between about 1 standard cubic centimeter per minute (sccm) to about 1000 sccm and in some embodiments from about 10 sccm to about 500 sccm. The flow rate can be constant through the processing step, although the flow rate and the composition of the gas can be varied systematically over time during processing, if desired. Alternatively, a static gas atmosphere can be used.

### 1. Carbon Particles

Traditional carbon black particles are considered amorphous since they do not produce x-ray diffractograms indicative of long-range order. Graphitization of carbon occurs at roughly 3,000°C, which is higher than the temperature of carbon black formation. However, carbon black has graphene sheets, which are curved forms of sp²-hybridized carbon. The graphene sheets are similar to graphite sheets except that they are curved. Thus, while the short and possibly intermediate range order of a graphene sheet may be similar to a graphite crystal structure, the curving of the graphene sheet prevents the formation of longer-range order. In addition, the nature of graphine sheets gives carbon black significantly different properties relative to graphite, such as with respect to their behavior in battery applications. Similar curved carbon sheets with short range and possible medium range order are found in bucky balls, i.e., fullerenes, and carbon nanotubes, such as single wall and multiple wall nanotubes.

Partial graphite formation of carbon black particles can occur at roughly temperatures of 2,000°C and higher. If the heating conditions are performed at gentle enough conditions, the particles do not sinter. Thus, the graphitic character of the particles can be increased without destroying the small particle size. Carbon particle formed by laser pyrolysis are observed to have less short-range order than traditional carbon blacks. See Bi et al., J. Materials Research, Vol. 10 (11), pp 2875-2884 (Nov 1995) entitled "Nanoscale carbon blacks produced by CO₂ laser pyrolysis," incorporated herein by reference. This article also describes the partial graphitization of laser black, i.e., carbon black formed by laser pyrolysis, using a heat treatment.

Carbon black particles, especially particles formed by laser pyrolysis, can be heat treated to form well faceted graphitized forms. The presence of greater disorder in laser black may facilitate the formation of graphite layers in a heat treatment step. These graphitized forms are desirable for use in battery applications in which the intercalation of lithium is more effective with graphite layers than curved graphene sheets. Desirably graphitized carbon particles can have a reproducible sharp peak at about 2θ = 26 degrees in a Cu K_{α}, x-ray diffractogram. In some embodiments of interest, graphitic domains occupy a majority of the volume of the particle. Furthermore, graphitized carbon nanoparticles can have a graphitic shell with a domain thickness in some embodiments of at least about 3.5 nm, in further embodiments at least about 5 nm and in further embodiments at least about 8 nm. A person of ordinary skill in the art will recognize that additional ranges of graphite shell thickness within the explicit ranges are contemplated and are within the present disclosure. The graphite shell thickness can be evaluated by visual examination of a transmission electron micrograph, as described in the Bi et al. article above.

In some embodiments, it is desirable for at least about 10 mole percent of the carbon to be in a graphitic environment, in further embodiments at least about 25 mole percent and in other embodiments at least about 50 mole percent. The degree of graphitic carbon can be estimated from infrared spectroscopy based on the different bonding and corresponding vibrational modes of the graphitic carbon relative to the amorphous carbon. Specifically, the mole percentages can be estimated using the ratio of the Raman scattering peaks at roughly 1600 wave numbers (cm⁻¹) with the peak at 1375 wave numbers. These peaks can be normalized using commercial graphite and fullerenes as standards. Alternatively, the carbon particles can be at least 10 volume percent graphitic, in other embodiments at least about 25 volume percent graphitic and in further embodiments at least about 50 volume percent graphitic. The volume percent graphite can be estimated from transmission electron micrographs. A person of ordinary skill in the art will recognize that additional ranges of graphitic content within the explicit ranges are contemplated and are within the present disclosure.

The heat treatment of the carbon particles can be performed, for example, in the apparatus shown in Fig. 11, as described above. An inert atmosphere can be used, and oxygen should be effectively excluded. A suitable inert gas is Ar. Generally, the heat treatment of the carbon nanoparticles is performed at temperatures of at least about 1,500°C, in other embodiments from about 1,800°C to about 3,200°C, in further embodiments from about 2,000°C to about 3,100°C and in additional embodiments from about 2,200°C to about 3,000°C. The heating generally is performed for at least about 5 minutes, in other embodiments from about 10 minutes to about 10 hours, and in further embodiments from about 30 minutes to about 4 hours. A person of ordinary skill in the art will recognize that additional ranges of heating temperatures and times within the explicit ranges are contemplated and are within the present disclosure.

### 2. Fluorination of Carbon Particles

Fluorine can be introduced into the carbon particles during their synthesis by laser pyrolysis or following synthesis of the particles using a fluorination agent. The amount of fluorine should be controlled since excess fluorine can result in the formation of gaseous products and the corresponding destruction of the carbon solid. Generally, the stoichiometry of the resulting carbon fluoride has a F/C ratio in the range from about 0.001 to about 1.9, in other embodiments from about 0.1 to about 1.7, in further embodiment from about 0.25 to about 1.5 and in additional embodiments from about 0.5 to about 1.1. The F/C ration can be approximately 1 in some embodiments. Expressed another way, the carbon fluoride can be expressed as (CFₓ) where x falls within the range 0 > x ≥ 2, in further embodiments in the range of 0.1 ≥ x ≥ 1.9, in some embodiments in the range of 0.5 ≥ x ≥ 1.5, and in additional embodiments in the range of 0.5 ≥ x ≥ 1.1 or in the range of 1.1 ≥ x ≥ 1.4. A person of ordinary skill in the art will recognize that additional ranges of fluorinated carbon composition within the explicit ranges are contemplated and are within the present disclosure. A person of ordinary skill in the art will recognize that additional ranges of F/C ratios within the explicit ranges are contemplated and within the present disclosure.

Carbon fluoride decomposes at higher temperatures so that the carbon fluoride particles are generally heated to temperatures less than about 500°C, in other embodiments from about 150°C to about 450°C and in further embodiments from about 250°C to about 375°C. This heating can generally be performed using the same apparatus and for the same times as described above. A person of ordinary skill will recognize that additional ranges of temperature within the explicit ranges are contemplated and are within the present disclosure.

In some embodiments, the fluorination reaction is performed after particle formation. Generally, the fluorination can be performed simultaneously with a heat treatment to increase graphite content or following heat treatment to increase graphite content. Heat treatment to increase graphite content is described above. If the fluorination is performed during this step, the atmosphere in the heating chamber can comprise fluorine gas optionally diluted with Ar or other inert gas. The gas pressure and other reaction parameters can be adjusted to get desired incorporation of fluorine into the particles. In particular, the heating and especially the cooling rate can be adjusted empirically to obtain the desired fluorination.

Fluorination of the particles after a heat treatment step to increase graphite content generally is performed at a temperature from about 200°C to about 600°C and in further embodiments from about 300°C to about 500°C. In these embodiments, the atmosphere in the reactor can be at atmospheric pressure or slightly above. The heating times generally are from about 3 hours to about 40 hours and in other embodiments from about 5 hours to about 30 hours. A person of ordinary skill in the art will recognize that additional ranges of temperatures and time within the explicit ranges above are contemplated and are within the present disclosure.

### C. Properties of the Particles

A collection of particles of interest generally has an average diameter for the particles of less than about 1 micron, alternatively less than about 500 nm, in other embodiments from about 2 nm to about 100 nm, alternatively from about 5 nm to about 75 nm, and in further embodiments from about 5 nm to about 50 nm. A person of ordinary skill in the art will recognize that additional ranges of particle diameters within the explicit ranges above are contemplated and are within the present disclosure. Particle diameters generally are evaluated by transmission electron microscopy. Diameter measurements on particles with asymmetries are based on an average of length measurements along the principle axes of the particle. Generally, the description of the particles refer to primary particles, which have little, if any, hard fusing between the primary particles, such that the secondary particles are essentially equivalent to the primary particles. In other words, the term particles, as used herein, refer to dispersible physical particles.

The particles produced by laser pyrolysis usually have a roughly spherical gross appearance. Specifically, crystalline particles tend to exhibit growth that is roughly equal in the three physical dimensions to give a gross spherical appearance. Amorphous particles generally have an even more spherical aspect. After heat treatment the particles may take non-spherical shapes reflecting the crystal lattice. Upon closer examination, crystalline particles generally have facets corresponding to the underlying crystal lattice.

Because of their small size, the particles tend to form loose agglomerates due to van der Waals and other electromagnetic forces between nearby particles. These agglomerates can be dispersed to a significant degree, for example, in an appropriate solution. Even though the particles form loose agglomerates, the submicron or nanometer scale of the particles is clearly observable in transmission electron micrographs of the particles. Furthermore, the particles can manifest unique properties due to their small size and large surface area per weight of material. For example, vanadium oxide nanoparticles can exhibit surprisingly high energy densities in lithium batteries, as described in U.S. Patent No. 5,952,125 to Bi et al., entitled "Batteries With Electroactive Nanoparticles," incorporated herein by reference.

The particles can have a high degree of uniformity in size. Laser pyrolysis, as described above, generally results in particles having a very narrow range of particle diameters. Furthermore, heat processing under suitably mild conditions does not alter the very narrow range of particle diameters. With aerosol delivery of reactants for laser pyrolysis, the distribution of particle diameters is particularly sensitive to the reaction conditions. Nevertheless, if the reaction conditions are properly controlled, a very narrow distribution of particle diameters can be obtained with an aerosol delivery system. As determined from examination of transmission electron micrographs, the particles generally have a distribution in sizes such that at least about 95 percent, and in further embodiments 99 percent, of the particles have a diameter greater than about 40 percent of the average diameter and less than about 225 percent of the average diameter. In some embodiments, the particles have a distribution of diameters such that at least about 95 percent, and in further embodiments 99 percent, of the particles have a diameter greater than about 45 percent of the average diameter and less than about 200 percent of the average diameter. A person of ordinary skill in the art will recognize that additional ranges of particle uniformity within the explicit ranges above are contemplates and are within the present disclosure.

Furthermore, in some embodiments effectively no particles have an average diameter no more than about 5 times the average diameter, in further embodiments 4 times the average diameter, and in additional embodiments 3 times the average diameter. In other words, the particle size distribution effectively does not have a tail indicative of a small number of particles with significantly larger sizes. This is a result of the small reaction region and corresponding rapid quench of the particles. An effective cut off in the tail of the size distribution indicates that there are less than about 1 particle in 10⁶ have a diameter greater than a specified cut off value above the average diameter. Narrow size distributions, lack of a tail in the distributions can be exploited in a variety of applications. A person of ordinary skill in the art will recognize that additional ranges of cut offs in the particle distribution within the explicit ranges are contemplated and are within the present disclosure.

In addition, the submicron particles produced by the techniques described herein generally have a very high purity level. The particles produced by the above described methods are expected to have a purity greater than the reactants because the laser pyrolysis reaction and, when applicable, the crystal formation process tends to exclude contaminants from the particle. Certain impurities on the surface of the particles may be removed by heating the particles to achieve not only high crystalline purity but high purity overall.

### D. Electrode and Cell Structures

A generic embodiment of a cell 650 is shown schematically in Fig. 12. Cell 650 has a negative electrode 652, a positive electrode 654 and separator 656 between negative electrode 652 and positive electrode 654. A single battery can include multiple positive electrodes and/or multiple negative electrodes within a single cell or within a plurality of cells. Electrolyte can be supplied in a variety of ways as described further below. Cell 650 generally comprises current collectors 658, 660 associated with negative electrode 652 and positive electrode 654, respectively. Multiple current collectors can be associated with each electrode if desired. Battery structures are described further below that are particularly suitable for high rate and medium rate applications, especially for implantable medical devices.

Lithium has been extensively used in primary and secondary batteries. An attractive feature of metallic lithium is that it is the most electropositive metal. Certain forms of metal, metal oxides and mixed metal oxides are known to incorporate lithium ions into its structure through intercalation or similar mechanisms such as topochemical absorption. Furthermore, intercalation of lithium ions can take place in suitable forms of a graphite lattices and carbon fluoride lattices.

In particular, lithium intercalates into the lattice of a positive electrode material during discharge of the battery. The lithium leaves the lattice upon recharging, i.e., when a voltage is applied to the cell such that electric current flows into the positive electrode due to the application of an external EMF to the battery. Positive electrode 654 acts as a cathode during discharge, and negative electrode 652 acts as an anode during discharge of the cell. Carbon fluoride particles can be used directly in a positive electrode for a lithium-based battery to provide a cell with a high energy density. Appropriate carbon fluoride particles can be an effective electroactive material for a positive electrode in either a lithium or lithium ion battery.

Positive electrode 654 can comprise electroactive nanoparticles, such as carbon fluoride particles, held together with a binder such as a polymeric binder. Particles for use in positive electrode 654 generally can have any shape, e.g., roughly spherical particles or elongated particles. For high rate and/or pulsed applications, metal vanadium oxides have been found to have good performance, and silver vanadium oxide can be used alone or combined with carbon fluoride electroactive particles.

Carbon fluorides are desirable materials for medium rate and/or low rate applications due to their very high energy density. These can be combined in various ways with high rate materials, such as silver vanadium oxide particles, in a battery for providing excellent performance in both the high rate and medium/low rate domains, as described further below. Generally, batteries incorporating metal vanadium oxide compositions in the positive electrode (cathode) are used as primary batteries.

While some electroactive materials are reasonable electrical conductors, a positive electrode generally includes electrically conductive particles in addition to the electroactive nanoparticles. The binder generally also holds these supplementary, electrically conductive particles. Suitable electrically conductive particles include conductive carbon particles, such as graphite, carbon black, metal particles such as silver particles, metal fibers such as stainless steel fibers, and the like. The graphite can have a BET surface area of at least 50 m²/g, in some embodiments at least about 100 m²/g, in further embodiments at least about 150 m²/g and in additional embodiments at least about 200 m²/g. A person of ordinary skill in the art will recognize that additional ranges of surface area within the explicit ranges are contemplated and are within the present disclosure.

High loadings of particles can be achieved in the binder. Particles can make up greater than about 80 percent by weight of the positive electrode, and in some embodiments at least about 90 percent by weight of the positive electrode. The binder can be any of various suitable polymers such as polyvinylidene fluoride, polyethylene oxide, polyethylene, polypropylene, polytetrafluoro ethylene, polyacrylates, ethylene-(propylene-diene monomer) copolymer (EPDM) and mixtures and copolymers thereof.

In some embodiments, the production of positive electrodes/cathodes involves the mixing together of the electroactive powders, the electrically conductive powders and the polymer and subsequent pressing of the materials at high pressure to form a cathode. In alternative embodiments, sufficient solvent is added to provide for blending of the mixture with a homogenizer or the like. An example of a suitable homogenizer is a T25 Basic Ultra-TURRAX Laboratory Dispenser/Homogenizer from IKA Works, available from VWR Scientific, San Francisco, CA. Homogenizers are known in the art to operate at high shear compared with other mixing approaches.

Using a homogenizer, it has been observed that better dispersion of the particles can be obtained. In some embodiments, the mixture is blended at appropriate speeds for about 1 minute to about 20 minutes, in further embodiments for about 2 minutes to about 10 minutes, and in other embodiments from about 2 minutes to about 5 minutes. High shear homogenizing can be conducted at greater than about 5000 rpm, and generally at about 8,000 rpm to about 24,000 rpm, which correspond to low settings on standard homogenizers. Homogenizing at higher rpm would be expected to yield similar results. Mixing in the homogenizer provides an extremely well dispersed blend of the components. Following mixing in the homogenizer, the mixture can be filtered, kneaded and rolled into a cathode sheet. The cathode is cut into a desired shape and then dried to remove the solvent. The drying can be performed in an oven, such as a vacuum oven. After drying, the cathode can be pressed, generally under pressures of, for example, about 3 to about 3.5 tons per cm². Following pressing of the cathode material, the cathode can be stored in a dry environment.

In the case of lithium batteries, the negative electrode can comprise lithium metal or lithium alloy metal either in the form of a foil, grid or metal particles in a binder. Lithium ion batteries generally use particles of a composition that can intercalate lithium. The particles are held with a binder in the negative electrode. Suitable intercalation compounds include, for example, graphite, synthetic graphite, coke, mesocarbons, doped carbons, fullerenes, tin alloys, SnO₂ and mixtures and composites thereof.

Current collectors 658, 660 facilitate flow of electricity from battery 650. Current collectors 658, 660 are electrically conductive and generally made of metal such as nickel, stainless steel, tantalum, titanium, aluminum, and copper and can be metal foil or preferably a metal grid. Current collector 658, 660 can be on the surface of their associated electrode or embedded within their associated electrode.

Separator element 656 is electrically insulating and provides for passage of ions. Ionic transmission through the separator provides electrical neutrality throughout the cell. The separator prevents electroactive compounds in the positive electrode from contacting electroactive compounds in the negative electrode, which would result in a short circuit.

A variety of materials can be used for the separator. For example, the separator can be formed from glass fibers that form a porous matrix. In some embodiments, separators can be formed from polymers such polyethylene and polypropylene. Suitable commercial polymer separators include Celgard from Hoechst Celanese, Charlotte, NC. Polymer separators are porous to provide for ionic conduction. Alternatively or additionally, polymer separators can be solid electrolytes formed from polymers such as polyethylene oxide. Solid electrolytes incorporate electrolyte into the polymer matrix to provide for ionic conduction with or without the need for liquid solvent.

Electrolytes for lithium batteries or lithium ion batteries can include any of a variety of lithium salts. Appropriate lithium salts generally have chemically inert anions. Suitable lithium salts include, for example, lithium hexafluorophosphate, lithium hexafluoroarsenate, lithium bis(trifluoromethyl sulfonyl imide), lithium trifluoromethane sulfonate, lithium tris(trifluoromethyl sulfonyl) methide, lithium tetrafluoroborate, lithium perchlorate, lithium tetrachloroaluminate, lithium chloride and combinations thereof.

If a liquid solvent is used to dissolve the electrolyte, the solvent generally is inert and does not dissolve the electroactive materials. Appropriate solvents can include, for example, propylene carbonate, dimethyl carbonate, diethyl carbonate, 2-methyl tetrahydrofuran, dioxolane, tetrahydrofuran, methyl ethyl carbonate, dipropyl carbonate, ethylene carbonate, γ-butyrolactone, dimethyl sulfoxide, acetonitrile, formamide, dimethyl formamide, triglyme (tri(ethylene glycol) dimethyl ether), diglyme (diethylene glycol dimethyl ether), DME (glyme or 1,2-dimethoxyethane or ethylene glycol dimethyl ether), nitromethane, and mixtures thereof.

Some embodiments involve the production of high rate batteries. Improved rate performance has been found with the use of highly ion conductive solvents for forming the electrolytes. Particularly suitable solvents include a mixture of DME with another solvent, in particular an alkylene carbonate. For example, one preferred mixture is a approximate 1:1 volume ratio of DME and ethylene carbonate or propylene carbonate. Generally, suitable solvents include, for example, from about 25 volume percent DME to about 75 percent DME and more preferably from about 33 volume percent to about 66 volume percent DME with the remainder being an alkylene carbonate.

The shape of the battery components can be adjusted to be suitable for the desired final product, for example, a coin cell, a rectangular construction or a cylindrical battery. The battery generally comprises a casing with appropriate terminals in electrical contact with current collectors and/or electrodes of the battery. If a liquid electrolyte is used, the casing should prevent the leakage of the electrolyte. The casing can help to maintain the battery elements in close proximity to each other to reduce resistance, i.e., impedance, within the battery, with respect to both appropriate electrical and ionic conductivity pathways. A plurality of battery cells can be placed in a single case with the cells connected either in series, in parallel and/or in an independent configuration.

### E. Battery Structures

In general, the size of the battery is selected to be convenient for the particular application. The selection of the size of the battery can involve a balance between providing a desired specific current capacity and/or specific energy while keeping the size of battery small. For use in implantable medical devices, it generally is desirable to keep the size small such that the medical device itself can be small.

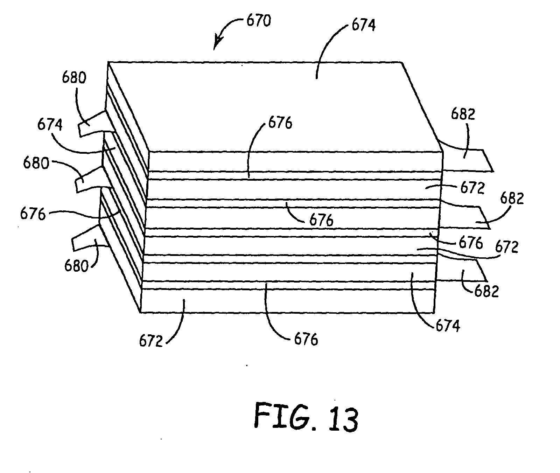

For some applications, such as for implantable medical devices, it may be desirable have a higher capacity while having constraints on the shape and dimensions of the resulting battery due to the constraints on the implantable device. Therefore, it may be desirable to incorporate particular battery designs. In particular, for appropriate embodiments to maintain the high rate or medium rate characteristics, it is desirable to maintain the thickness of the cathode within a certain range. However, it may be desirable to keep the thickness of the battery below certain values such that a circularly wound battery may not be desirable. Suitable battery designs, which can have a selected total cathode volume while limiting the thickness of the battery, are shown in Figs. 13 to 14. These battery structures correspond, respectively, to stacked electrodes and a structure with a serpentine anode having individual cathode plates between the folds of the anode. Batteries with serpentine anodes are described further in U.S. Patent No. 4,830,940 to Keister et al., incorporated herein by reference.

Referring to Fig. 13, battery 670 comprises three anode elements 672, three cathode elements or plates 674 and five separators 676, with the electrodes stacked in parallel. A separator 676 is placed between each adjacent anode 672 and cathode 674. Since the separator electrically isolates an anode from a cathode, separator 676 may extend slightly beyond the surfaces of one or more of the corresponding electrodes to ensure appropriate separation of the electrodes. The number of each type of electrode can be selected to yield the desired size of the battery and battery capacity, which depend also on thickness of the electrodes. Cathode thickness is discussed further below. In some high rate embodiments, the battery can comprise at least 6 cathode elements and in further embodiments, the battery can comprise at least 8 cathode elements. Other higher or lower number of cathode elements can be used as appropriate to achieve desired battery parameters.

While battery 670 is shown with an equal number of anode elements 672 and cathode elements 674, the number of elements can be different, such as having an additional cathode element 674 by placing another separator 676 and cathode element 674 at the bottom of the structure in Fig. 13 or such as having an additional anode element 672 by placing another separator 676 and anode element 672 at the top of the structure in Fig. 13. Generally, an anode element 672 and/or a cathode element 674 can be associated with a current collector, such as a current collector as described above. An anode element 672 and/or a corresponding current collector can have a tab 680 for connection of the battery with an external circuit, and similarly a cathode element 674 and/or a corresponding current collector can have a tab 682. Tabs associated with cathode elements 674 are connected in parallel, and tabs associated with anode elements 672 are similarly connected in parallel. The battery elements generally are placed within a suitable case with electrolyte such that anode elements 672 and cathode elements 674 are respectively connected to electrical contacts for connecting the battery to an external circuit.

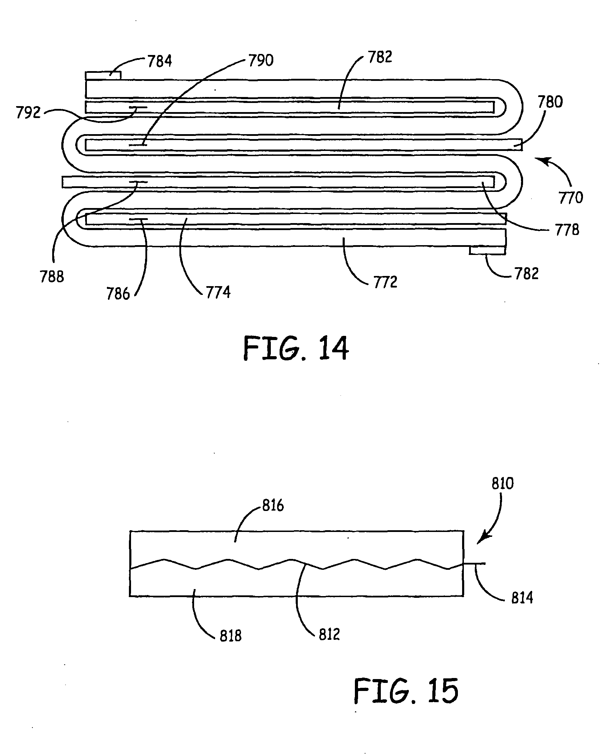

Referring to Fig. 14, battery 770 comprises a serpentine anode 772 and cathode plates 774, 776, 778, 780. For this serpentine construction, various separator embodiments can be used individually or combined. For example, a serpentine separator can be used that winds on both sides of anode 772. Alternatively, cathode plates 774, 776, 778, 780 can be wrapped in separator. As shown in Fig. 14, anode 772 comprises conducting tabs 782, 784 to provide electrical contact with the anode. In some embodiments, tabs 782, 784 can be in electrical connection with a conductive case that serves as the terminal for the anode. Cathode plates 774, 776, 778, 780 comprise conductive tabs 786, 788, 790, 792 for connection to a cathode terminal. Additional conductive tabs or conductive tabs at other locations can provide electrical connections as desired. Battery 770 is shown with four turns and four cathode plates, although a greater or lesser number of turns and corresponding cathode plates can be used as appropriate for a particular application.

In general, these battery structures are placed within a case along with an appropriate electrolyte and sealed. Prior to sealing, the conductive tabs are connected to corresponding terminals. A conductive case can serve as one terminal with an appropriate insulating spacer separating the case form other terminals. The terminals then provide for discharge of the battery.

The high rate capable batteries described herein and in other applications incorporated herein are especially useful in the production of certain implantable medical devices, in particular defibrillators. Defibrillators provide pulses of electricity to a patient's heart to induce regular beating. Lithium batteries incorporating silver vanadium oxide have found important commercial use in the production of implantable defibrillators. For use in defibrillators, the battery cells deliver high current pulses in rapid succession.

Defibrillators generally have other functions. For example, an implantable defibrillator has a monitoring function such that it can sense when a patient's heart undergoes fibrillation. In addition, combination pace makers and defibrillators can be constructed. Combination implantable devices can include a separate battery, such as a lithium iodide battery or carbon monofluoride battery, to perform the ongoing pacing operations such that the high rate silver vanadium oxide battery could be reserved for pulsed operation without depleting the battery. In addition to implantable medical devices with pacing and/or defibrillating functions, the improved batteries described herein can be suitable for cardiac resynchronization therapy devices for heart failure, ventricular assist devices, neurostimulators, combinations thereof and with pace makers and defibrillators and the like.

The submicron carbon fluoride and/or carbon particles can be used for implantable medical devices with pacing functions, monitoring functions or other functions that are suitable for medium rate or low rate applications. These materials can be combined with a high rate material or other medium or low rate materials to serve appropriate functions or combination of functions, such as end of life indications or monitoring functions combined with defibrillating functions. Of course, in forming an electrode structure, the electroactive materials generally can be combined with a binder, electrically conductive particles, such as those described above, as well as minor amounts of other additives. These possible generic components are not discussed with respect to specific structures to simplify the discussion, although a person of ordinary skill in the art will recognize that generic components of an electrode can be included within the electrode structures with the electroactive compositions. Regardless of the particular purposes, various structures can be used to combine a plurality of different electroactive materials. For example, the two electroactive materials are combined within a composite electrode material.

For some embodiments, it may be desirable to combine materials that perform well in pulsed operation, i.e., a high rate material such as silver vanadium oxide, with a high capacity material, such as carbon fluoride. Specifically, it can be advantageous to place a layer of cathode active material on opposite sides of a current collector that is formed from stainless steel, titanium, aluminum, tantalum or a combination thereof. Such a cathode structure is shown schematically in Fig. 15. Cathode structure 810 comprises a current collector 812 having a connection tab 814, a layer of first electroactive composition 816, such as carbon fluoride, and a layer of second electroactive composition 818, such as silver vanadium oxide. While the structure in Fig. 15 can have desirable performance properties, various other layer structures are possible to take advantage of the properties of a plurality.of electroactive materials.

The testing protocol for high rate batteries is described in detail in U.S. Patent No. 6,503,646 to Ghantous et al., entitled "High Rate Batteries, incorporated herein by reference, and U.S. Application Serial No. 10/624,226, filed on July 22, 2003 to Ghantous et al., entitled "High Capacity And High Rate Batteries," incorporated herein by reference. A suitable testing protocol for medium rate batteries can apply a constant current pulse train to the cell with a wait time between pulses within the train.

The embodiments described above are intended to be illustrative and not limiting. Additional embodiments are within the claims below. Although the present invention has been described with reference to preferred embodiments, workers skilled in the art will recognize that changes may be made in form and detail without departing from the spirit and scope of the invention.

## Claims

1. A collection of particles comprising graphitic carbon fluoride with an average formula (CFₓ) with 1.9 ≥ x ≥ 0.6 and having an average particle diameter of no more than 1 µm (micron), wherein the collection of particles comprises particles having a graphitic shell with a domain thickness of at least about 3.5 nm.

2. The collection of particles of claim 1, having an average particle size no more than about 500 nm, preferably having an average particle size of no more than about 100 nm.

3. The collection of particles of claim 1 or claim 2, wherein the graphitic carbon fluoride has an average formula (CFₓ) with 1.6 ≥ x ≥ 0.75, preferably wherein the graphitic carbon fluoride has an average formula (CFₓ) with 1.5 ≥ x ≥ 0.85.

4. The collection of particles of any of claims 1 to 3, wherein graphitic carbon fluoride particles have a graphitic shell with a domain thickness of at least about 5 nm, wherein graphitic carbon fluoride particles have a graphitic shell with a domain thickness of at least about 8 nm.

5. The collection of particles of any of claims 1 to 4, wherein at least about 95 percent of the primary particles have a diameter greater than about 45 percent of the average diameter and less than about 200 percent of the average diameter

6. The collection of particles of any of claims 1 to 5, wherein essentially no primary particles have a diameter greater than about 4 times the average diameter.

7. An electrochemical cell comprising:
a) an anode;
b) a cathode comprising a particle collection of any of claims 1 to 6; and
c) an electrolyte activating the cathode and anode.

8. The electrochemical cell of claim 7, wherein the anode comprises elemental lithium metal.

9. The electrochemical cell of claim 7 or claim 8, where the electrolyte comprises 1,2-dimethoxyethane, propylene carbonate, dimethyl carbonate, ethylene carbonate, γ-butyrolactone and mixtures thereof and a lithium salt selected from the group consisting of LiBF₄, LiPF₆, LiAsF₆, LiClO₄, or mixtures thereof.

10. An implantable medical device comprising an electrochemical cell of any of claims 7 to 9, wherein the device is selected from the group consisting of neurostimulators, pacemakers, congestive heart failure devices and implantable cardioverter defibrillators.

11. A collection of particles comprising carbon fluoride with a formula of (CFₓ) with 1.9 ≥ x ≥ 0.6 and having an average particle diameter of no more than 1 µm (micron), wherein at least about 95 percent of the primary particles have a diameter greater than about 45 percent of the average diameter and less than about 200 percent of the average diameter.

12. The collection of particles of claim 11, having an average particle size no more than about 250 nm.

13. The collection of particles of claim 11 or claim 12, having an average formula (CFx) with 1.6 ≥ x ≥ 0.75.

14. The collection of particles of any of claims 11 to 13, wherein the graphitic carbon fluoride particles have a graphitic shell with a domain thickness of at least about 5 nm.

15. The collection of particles of any of claims 11 to 14, wherein essentially no particles have a diameter greater than about 4 times the average diameter.

16. A method for forming carbon fluoride, the method comprising heating carbon black particles to a temperature of at least about 1,500 °C and heating the particles in the presence of a fluorinating agent.

17. The method of claim 16, wherein the heating of the particles in the presence of a fluorinating agent is performed during the heat treatment at a temperature of at least about 1,500 °C, or wherein the heating of the particles in the presence of a fluorinating agent is performed after the heat treatment at a temperature of at least about 1,500 °C.

18. The method of claim 16 or claim 17, wherein the heating of the particles in the presence of a fluorinating agent is performed at a temperature from about 300 °C to about 600 °C.

19. The method of any of claims 16 to 18, wherein the fluorinating agent comprises HF, IF₅, F₂ or a combination thereof, and/or wherein the carbon black comprises acetylene black, and/or wherein the carbon black comprises carbon particles formed by reacting a reactant stream comprising carbon precursors, the reaction driven by an electromagnetic radiation source.

20. The method of any of claims 16 to 19, wherein the carbon fluoride has an average particle size of no more than 1 µm (micron), preferably wherein the carbon fluoride has an average particle size of no more than about 250 nm.

21. The method of any of claims 16 to 20, wherein the carbon fluoride has a graphitic shell with a domain thickness of at least about 3.5 nm.

22. The method of any of claims 16 to 21, wherein the carbon fluoride has a formula of (CFₓ) with 1.9 ≥ x ≥ 0.01.

23. The method of any of claims 16 to 22, wherein the heating of the carbon black particles to a temperature of at least about 1,500 °C comprises heating the particles to at least about 1,800 °C.

24. A method for forming carbon fluoride particles, the method comprising reacting a flowing reactant stream comprising a carbon precursor and a fluorine precursor to form carbon fluoride particles wherein the reaction is driven by an electromagnetic radiation source.

25. The method of claim 24, wherein the degree of fluorination is selected to form CFₓ, with 1.9 ≥ x ≥ 0.01.

26. The method of claim 24 or claim 25, wherein the radiation source is an infrared laser.

27. The method of any of claims 24 to 26, wherein the carbon fluoride particles have an average diameter no more than about one µm (micron).

28. The method of any of claims 24 to 27, further comprising heating the particles to a temperature of at least about 1,500 °C.

29. The method of any of claims 24 to 28, wherein the carbon fluoride has a graphite shell with a domain thickness of at least about 3.5 nm.

30. A method for forming fluorinated carbon, the method comprising exposing carbon particles to a fluorinating agent, wherein the carbon particles were formed by laser pyrolysis.

31. The method of claim 30 wherein the fluorinating agent is HF, IF₅, F₂ or a combination thereof.

32. The method of claim 30 or claim 31, wherein the carbon particles have an average diameter no more than about 1 µm (micron).

33. The method of any of claims 30 to 32, wherein the carbon particles are further heated to temperatures of at least about 1,500 °C to form particles with a graphitic shell with a domain thickness of at least about 3.5 nm.

34. The method of any of claims 30 to 33, wherein the exposing of the particles to a fluorinating agent is performed at a temperature from about 300 °C to about 600 °C, or wherein the exposing of the particles to a fluorinating agent is performed at a temperature of at least about 1500 °C.

35. A battery comprising a lithium based anode, a cathode comprising heat-treated carbon black particles having a graphite shell having a domain thickness of at least about 3.5 nm and an electrolyte comprising lithium cations.

36. The battery of claim 35, wherein the anode comprises elemental lithium metal or a lithium metal alloy.

37. The battery of claim 35 or claim 36, wherein the carbon black particles have an average particle size of no more than about 1 µm (micron).

38. The battery of any of claims 35 to 37, wherein the carbon black particles comprise (CFₓ).
